# EUROPEAN PATENT SPECIFICATION

(11) **EP 2 712 555 B1**
(45) Date of publication and mention of the grant of the patent: **23.08.2017**
(21) Application number: 13199562.3
(22) Date of filing: 27.05.2008
(51) Int. Cl.: A61B 17/06

(54) **Suture packaging**
Fadenverpackung
Emballage de suture

(30) Priority: 29.05.2007 US 940625 P
(43) Date of publication of application: 02.04.2014
(62) Divisional of application: 08756330.0
(73) Proprietor: Ethicon LLC, 00969 Guaynabo (PR)
(72) Inventor: Hunter, William L., Vancouver, British Columbia V6A 1B6 (CA); Herrmann, Robert A., Vancouver, British Columbia V6B 1X9 (CA); Naimagon, Alexander, Richmond, British Columbia V6Y 1H3 (CA); Avelar, Rui, Vancouver, British Columbia V6J 2W7 (CA)
(74) Representative: Carpmaels & Ransford LLP

(56) References cited:
- EP-A1- 0 717 958
- DE-A1- 19 802 213
- US-A- 2 647 625
- US-A1- 2003 204 195

## Description

### FIELD OF THE INTENTION

The present invention relates generally to packaging for bidirectional sutures.

### BACKGROUND

Sutures typically consist of a filamentous suture thread attached to a needle with a sharp point (attachment of sutures and surgical needles is described in U.S. Patent Nos. 3,981,307, 5,084,063, 5,102,418, 5,123,911, 5,500,991, 5,722,991, 6,012,216, and 6,163,948, and U.S. Patent Application Publication No. 2004/0088003). Self-retaining sutures (often referred to as "barbed sutures") differ from conventional sutures in that they possess numerous tiny retainers (often referred to as "barbs") which anchor into the surrounding tissue following deployment, thereby eliminating the need to tie knots to affix adjacent tissues together and have been described in, for example, U.S. Patent No. 6,848,152 and European Patent 1 075 843. A self-retaining suture may be unidirectional, having one or more retainers oriented in one direction along the length of the suture thread; or bidirectional typically having one or more retainers oriented in one direction along a portion of the thread, followed by one or more retainers oriented in another (often opposite) direction over the remainder of the thread (as described in the context of barbed retainers in U.S. Patent Nos. 5,931,855 and 6,241,747). Although any number of sequential or intermittent configurations of retainers are possible, the most common form involves a needle at one end, followed by barbs projecting "away" from the needle until the transition point (often the midpoint) of the suture is reached; at the transition point the configuration of barbs reverses itself 180° (i.e., the barbs are now facing in the opposite direction) along the remaining length of the suture thread before attaching to a second needle at the opposite end.

In most suture packages, at least one end of the suture or, in the case of armed sutures (that is, those having a needle pre-loaded to at least one end of the suture), the needle, is often separated from the suture material to facilitate grasping that end of the suture or the needle (often with a surgical instrument such as a needle driver), whichever the case may be. Similarly, some suture packages separate each end of the armed or unarmed suture, thus facilitating grasping of each end or grasping the needle attached to each end. Most package designs feature a single housing where the suture material is contained; often coiled together or wound into an oval or "figure 8" conformation: see, for example, the suture package described in U.S. Patent No. 5,121,836. Such designs are suboptimal for a bidirectional self-retaining suture for severl reasons. First and foremost, the orientation of the retainers makes it likely that retainers oriented in one direction will interlock or "catch" those oriented in another direction if both sets are contained in the same housing. When pulling the suture from the package (typically by pulling on the needle with a needle driver) this will cause the suture barbs to grab or become stuck; often causing the suture to become entangled with itself. Second, even if the suture is successfully removed from the package, the same problem can still occur in the operating field. Since the suture material often has "memory" it may often coil even after removal from the packaging. Once again, the barbs of differing orientations can become entwined and locked together, slowing suturing or creating tangles that are problematic for the surgeon.

Other package designs directed to minimizing or eliminating points at which the packaged suture may intersect with itself include a molded spiral suture package described in U.S. Patent No. 5,154,283, a folded suture package described in U.S. Patent No. 6,659,70 comprising a series of subpanels superimposed upon one another, and various designs having series of perforations, tabs, or other guide elements for holding the suture in a particular configuration in the package: see, for example, U.S. Patent Nos. 6,938,755, 6,739,450, and U.S. Patent Application. Publication No. 2004/0050721. Whether or not any of these types of packaging are actually suited to reducing tangling of self-retaining sutures, they may entail increased manufacturing materials requirements and increased design complexity. EP0717958 discloses a package for retaining at least one ligature comprising a circular plate having a circular recessed base in the central portion thereof.

### SUMMARY

Suture packages may be configured to prevent or reduce catching or tangling of bidirectional sutures. The invention is defined in the independent claim. Preferred embodiments are defined in the dependent claims.

There is provided a package for releasably holding at least one suture with first and second segments, the package including a base having at least one surface and first and second suture holders for segregating the first and second segments, respectively, the first and second suture holders engaging the base. In one aspect the suture holders are housings having at least one aperture for the suture or suture to pass through. In another aspect the suture holders are guides, including, for example, spools. In yet another aspect, the suture holders share at least one segregating wall having a passage through which a suture may extend from one suture holder to the other In yet a further aspect the suture holders may include combinations of guides, housings, and segregating walls.

There is also provided a package for releasably holding at least one suture with a first segment having a plurality of retainers disposed proximally to a first end, and a second segment having a plurality of retainers disposed proximally to a second end, the package including a base having at least one surface and first and second suture holders for segregating the first and second segments, respectively, the first and second suture holders engaging the base. In one aspect, the suture holders are housing having at least one aperture for the suture or sutures to pass through. In another aspect, the suture holders are guides, including for example spools.
In yet another aspect, the suture holders share at least one segregating wall having a passage through which a suture may extend from one suture holder to the other. In yet a further aspect, the suture holders may include combinations of guides, housings, and segregating walls.

Each of the foregoing packages may further include a needle park disposed at or proximally to at least one of the first and second suture holders.

Each of the foregoing packages may also include at least in part a composition having a biological agent. The composition may have at least one biological agent selected from the class of anti-proliferative agents, anti-angiogenic agents, anti-infective agents, fibrosis-inducing (pro-scarring and/or pro-healing) agents, anti-scarring (fibrosis-inhibiting and/or anti-adhesion) agents, lubricious agents, echogenic agents, radio-opaque agents (and/or agents that enhance visualization under X-ray, CT, MRI and/or PET scanning) anti-inflammatory agents, cell cycle inhibitors, radioactive isotopes, analgesics, anesthetic agents, and anti-microtubule agents.

### BRIEF DESCRIPTION OF THE DRAWINGS

FIG. 1 is a plan view of an armed bidirectional suture with a needle attached to each end.
FIG. 2 is a plan view of a prior art suture package.
FIG. 3 is a plan view of an embodiment of a compartmented suture package in the closed position, according to the present disclosure.
FIG. 4a is a plan view of an embodiment of a compartmented suture package in the open position, according to the present disclosure.
FIG. 4b is a perspective view of an embodiment of a compartmented suture package in the open position, according to the present disclosure.
FIG. 5 is a plan view of another embodiment of a suture package with one housing in the open position and another housing in the closed position, according to the present disclosure.
FIG. 6 is a plan view of another embodiment of a compartmented suture package, according to the present disclosure.
FIG. 7 is a plan view of another embodiment.
FIG. 8a is a perspective view of one side of another embodiment of a compartmented suture package, according to the present disclosure.
FIG. 8b is a perspective view of another side of the suture package of FIG. 8a of a compartmented suture package, according to the present disclosure.
FIG. 9 is a plan view of an embodiment of a spooled suture package, according to the present disclosure.
FIG. 10 is a plan view of an embodiment of a spooled suture package, according to the present disclosure.
FIG. 11 is a plan view of another embodiment of a spooled suture package, according to the present disclosure.
FIG. 12 is a plan view of another embodiment of a spooled suture package, according to the present disclosure.
FIG. 13a is a plan view of another embodiment of a spooled suture package, according to the present disclosure.
FIG. 13b is a perspective view of another side of the spooled package of FIG. 13a.
FIG. 14a is a perspective view of another embodiment of a spooled suture dispenser, according to the present disclosure.
FIG. 14b is another perspective of the spooled suture package of FIG. 14a.
FIG. 14c is a perspective view of another embodiment of a spooled suture dispenser, according to the present invention.
FIGS. 14d and 14e are sectional views illustrating alternative groove shapes for the spooled suture dispenser of FIG. 14c.
FIG. 14f is a perspective view of another embodiment of a spooled suture dispenser, according to the present invention.
FIG. 14g is a perspective view of another embodiment of a spooled suture dispenser, according to the present invention.
FIG. 15 is a plan view of an embodiment of a spooled and compartmented suture package, according to the present disclosure.
FIGS. 16a and 16b are perspective views of the deployment of a bidirectional armed suture from an embodiment of a compartmented and spooled suture package, according to the present disclosure.
FIG. 17 is a perspective view of the deployment of an armed bidirectional suture from an embodiment of a compartmented suture package, according to the present disclosure.

### DETAILED DESCRIPTION

Prior to setting forth the invention, it may be helpful to an understanding thereof to first set forth definitions of certain terms that are used hereinafter

"Suture package" (or "package" or "dispenser") refers to a device for releasably holding at least one suture.

"Self-retaining system" refers to a self-retaining suture together with means for deploying the suture into tissue. Such deployment means includes, without (limitation, suture needles and other deployment devices as well as sufficiently rigid and sharp ends on the suture itself to penetrate tissue.

"Self-retaining suture" (or "barbed suture") refers to a suture that does not require a knot or a suture anchor at its end in order to maintain its position into which it is deployed during a surgical procedure. These may be monofilament sutures or braided sutures, and are positioned in tissue in two stages, namely deployment and affixation, and include at least one tissue retainer.

"Tissue retainer" (or simply "retainer" or "barb") refers to a suture element having a retainer body projecting from the suture body and a retainer end adapted to penetrate tissue. Each retainer is adapted to resist movement of the suture in a direction other than the direction in which the suture is deployed into the tissue by the surgeon, by being oriented to substantially face the deployment direction. As the tissue-penetrating end of each retainer moving through tissue during deployment faces away from the deployment direction (the direction of the passage of the suture during deployment), the tissue retainers should not catch or grab tissue during this phase. Once the self-retaining suture has been deployed, a force exerted in another direction, often substantially opposite to the deployment direction, to affix the suture in position causes retainers to be displaced from their deployment positions of resting substantially along the suture body and causes retainer ends to penetrate into the tissue resulting in tissue being caught between the retainer and the suture body.

"Suture thread" refers to the filament component of the suture, and, for sutures requiring needle deployment, does not include the needle.

"Suture deployment end" refers to an end of the suture to be deployed into tissue; one or both ends of the suture may be suture deployment ends. The suture deployment end may be attached to deployment means such as suture needles, or may be sufficiently sharp and rigid to penetrate tissue on its own.

"Armed suture" refers to a suture having a suture needle on at least, one suture deployment end.

"Bidirectional suture" refers to a self-retaining suture having retainers oriented in one direction at one end and retainers oriented in the other direction at the other end. A bidirectional suture is typically armed at each end of the suture thread.

"Transition segment" refers to a retainer-free portion of a bidirectional suture located between a first set of retainers oriented in one direction and a second set of retainers oriented in another direction.

"Suture needle" (or simply "needle") refers to a needle used to deploy sutures into tissue, which come in many different shapes, forms and compositions. There are two main types of needles, traumatic needles and traumatic needles. Traumatic needles have holes or eyes which are supplied separate from the suture thread and are threaded on site. Atraumatic needles are eyeless and are attached to the suture at the factory by swaging whereby the suture material is deformed to a final shape to hold the suture and needle together. Most modern sutures are swaged atraumatic needles. Suture needles are described, for example, in U.S. Patent Nos. 6,322,581 and 6,214,030 (Mani, Inc., Japan); and 5,464,422 (W.L. Gore, Newark, DE); and 5,941,899; 5,425,746; 5,306,288 and 5,156,615 (US Surgical Corp., Norwalk, CT); and 5,312,422 (Linvatec Corp., Largo, FL); 7,063,716 (Tyco Healthcare, North Haven, CT). Other suturing needles are described, for example, in U.S. Patent Nos. 6,129,741; 5,897,572; 5,676,675; and 5,693,072.

"Needle park" refers to a holder on which a needle of an armed suture is secured in the suture package.

The details of one or more embodiments are set forth in the description below. Other features, objects and advantages will be apparent from the description, the drawings, and the claims.

### SUTURES AND SINGLE-HOUSING PACKAGING

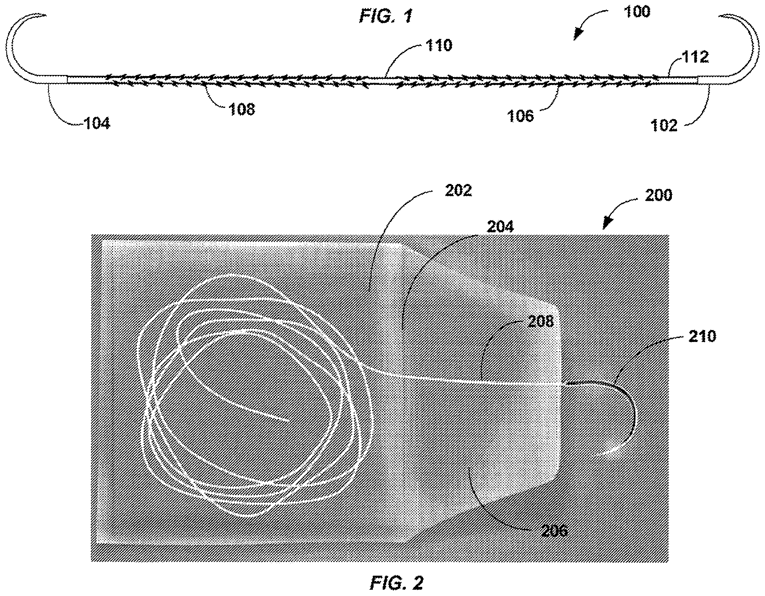

Referring first to FIGS. 1 and 2, an armed bidirectional suture 100 includes suture thread 112 attached to needle 102 at one end and needle 104 at the other end. A plurality of retainers 106 proximal to and facing the needle 102 is provided to enable suture 100 to resist movement in the direction that the tissue penetrating end of those retainers 106 face when suture 100 is deployed into tissue. Similarly, a plurality of retainers 108 proximal to and facing the needle 104 is provided to enable suture 100 to resist movement in the direction that the tissue penetrating end of those retainers 108 face when suture 100 is deployed into tissue. A conventional suture package 200 is shown in FIG. 2, containing a coiled-up plain (that is, retainer-free) suture 208 with a needle 210 and consisting of a single compartment or housing 202 having an open end 204 for removing suture 208. Flap 206 closes off open end 204. Package 200 is suitable for plain sutures such as suture 208, but it can be readily understood that placement of a bidirectional suture (armed or otherwise) in such a conventional package could be problematic, as retainers facing in opposite directions can catch on one another if a bidirectional suture is simply allowed to coil, bunch, or otherwise rest with retainers having opposing orientations coming into contact with one another.

### SEPARATION OF RETAINERS

When used with bidirectional sutures, the suture packages described herein provide segregation of each plurality of retainers oriented in a given direction, thereby preventing tangling of the retainers and facilitating removal of a suture from the package for use. While the examples herein describe the use of these suture packages with armed bidirectional sutures, it is to be understood that they may also be used with bidirectional sutures without needles, unidirectional sutures with or without needles, as well as plain sutures with or without needles. Furthermore, the suture packages described herein are not limited to housing only single sutures or only a particular number of needles; they can be used with any number of sutures (as long as, of course, retainers oriented in opposing directions are not grouped together) and any number of needles, to the extent that the suture packages' size or other physical parameters allow. Similarly, while the self-retaining sutures shown in the present examples are generally barbed, it is to be understood that the herein-described suture packages can be used with retainers of any configuration (for example, without limitation, frusto-conical retainers as disclosed in European Patent 1 075 843 and barbed retainers as disclosed in U.S. Patent No. 6,241,747).

The suture packages described herein generally include a base having at least one surface, and a configuration that segregates the two opposing retainer configurations from coming into contact with each other that in turn attaches to the base; this can include, without limitation, separate compartments for each retainer configuration or a spooling/dispensing mechanism that prevents the opposing retainers from contracting each other and becoming entangled. In one embodiment, the suture packages described herein generally include a base having at least one surface, and at least two suture holders for segregating portion of sutures from one another, the holders engaging the base. However, it is to be understood that the term "suture holder" can be used to designate an area of the suture package on which part of the suture is disposed but lacking any particular retention elements such as housings or guides; that is, as long as the portions of the suture can be releasably maintained in segregation from one another, the areas of the suture package that each segregated portion occupies can properly be described as a "suture holder". For instance, without limitation, a suture package as claimed herein can include only one housing (or compartment) as the single housing would be sufficient to segregate the two portions if only one portion were disposed within it. In a similar manner, a wall with some sort of aperture or discontinuity (to allow passage of the suture) disposed between the two portions to be segregated can achieve the same end, by effectively defining suture holders on either side of that wall.

As bidirectional sutures can be maintained in these suture packages so that tissue retainers facing in opposing directions are segregated from one another, a portion of suture having retainers facing in a particular direction can be disposed in one suture holder while a portion of suture having retainers facing in an opposing direction can be disposed in the other suture holder. Thus, the connecting portion of the suture between each of the two portions disposed in the suture holders would lie somewhere between the suture holders, and would therefore include the retainer-free transitional segment. The suture holders may be disposed on the same surface of the base such that the base spans from one holder to the other, or they may be disposed on different surfaces of the base. In either case, the suture may extend from one holder to the other anywhere on the base, above the base, or through the base. Similarly, where the base can be described as having a single surface (for example, without limitation, a spherical or egg-shaped base) such that the suture holders are by definition on the same surface of the base, the connecting portion of suture may continue along (or above) the surface of the base or may run at least in part through the base. The base is not limited to being a single piece, and may instead include one or more pieces connected together.

### A. Housings

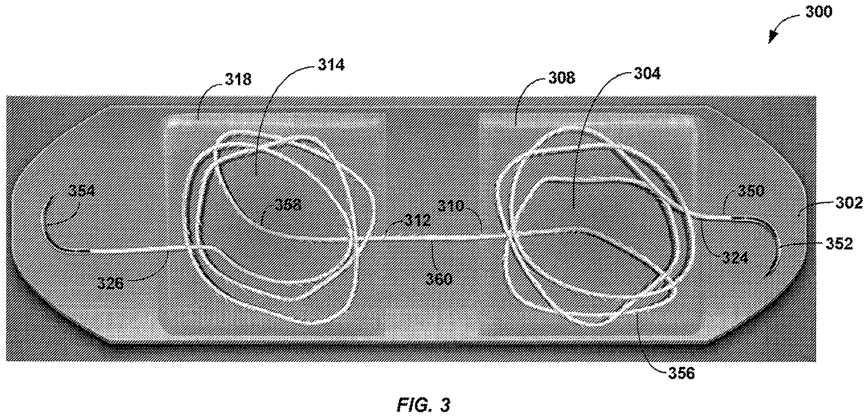

Referring now to FIG. 3, suture package 300 releasably secures suture 350. Suture package 300 includes base 302 on which are disposed housings 304 and 314. Housing 304 includes cover 308 and houses a portion of suture 350 having a plurality of retainers 356 oriented in one direction, while housing 314 includes cover 318 and houses a portion of suture 350 having a plurality of retainers 358 oriented in a substantially opposite direction to retainers 356. Covers 308 and 318 are affixed directly to the base around the covers' respective edges, except at various points and therefore allow passage of suture 350, either between the housings or out of the housings, through such discontinuities or apertures in the attachment between the covers 308 and 318 and base 302. As such, a portion of suture 350, including transitional segment 360, rests between the two housings, passing out of housings 304 and 314 through apertures 310 and 312, respectively. Similarly, suture needles 352 and 354 remain outside housings 304 and 314, having run through apertures 324 and 326.

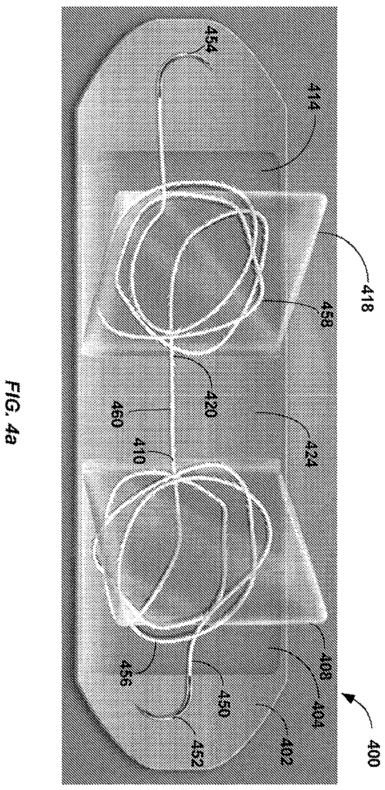

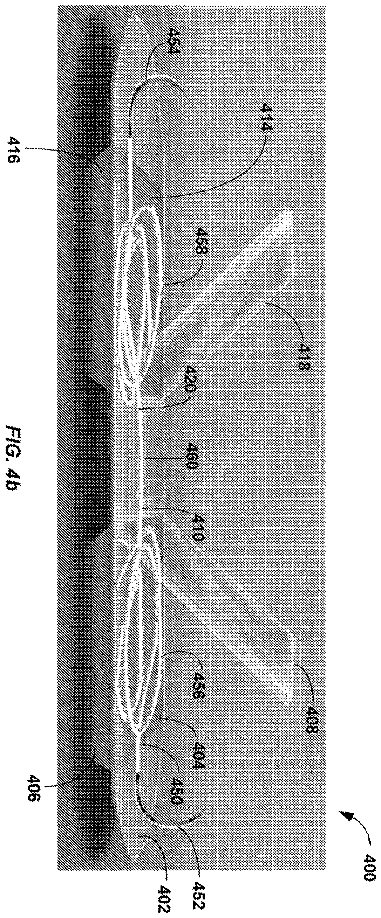

FIGS. 4a and 4b depict a suture package 400 releasably securing suture 450, package 400 having housings 404 and 414 in the open position on base 402. While covers 408 and 418 along one edge of each such cover (thereby opening in a hinged fashion) with apertures 410 and 420 to allow extension of transitional segment 460 of suture 450 to rest along base 402 between the two housings, the portions of the base 402 covered by covers 408 and 418 are recessed into wells 406 and 416, respectively. These wells 406 and 416 provide a greater volume in the housings 404 and 414 for each of the suture segments 456 and 458 to rest and thus do not press the suture 450 into a flattened coiled configuration, further reducing the likelihood of the suture retaining "memory" of its coiled configuration in the suture package 400 once the suture 450 is dispensed for deployment into tissue. As in the example shown in FIG. 3, suture needles 452 and 454 suture remain outside housings 404 and 414.

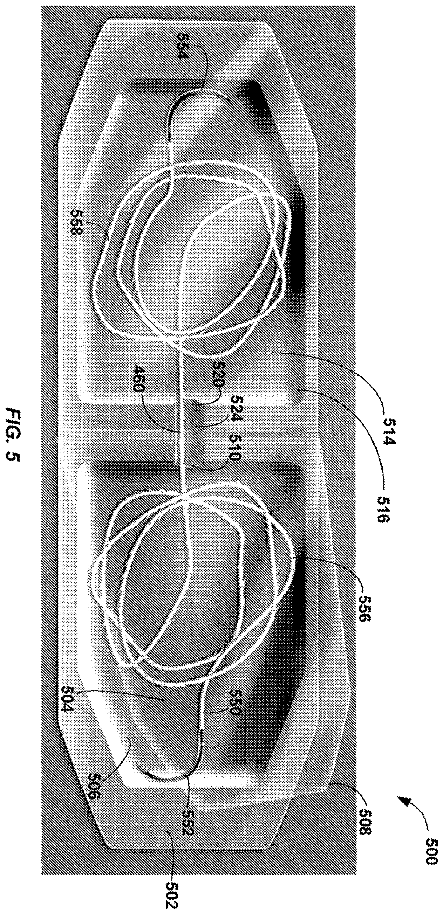

While the two preceding examples describe separate covers for each housing in a two-housing suture package, suture packages having two housings may be provided with a single cover spanning both housings. With reference to FIG. 5, suture package 500 releasably secures suture 550. Suture package 500 includes base 502 on which are disposed housings 504 and 514 having wells 506 and 516, respectively, and single cover 508 extending over both housings. Housing 504 (over which cover 508 is shown in the open position) houses a portion of suture 550 having a plurality of retainers 556 oriented in one direction as well as needle 552 attached to an end of the suture 550. Housing 514 includes well 516 recessed into base 502, which is shown as closed by the portion of cover 508 spanning it and houses a portion of suture 550 having a plurality of retainers 558 oriented in a substantially opposite direction to retainers 556. Needle 554 is also enclosed within housing 514. While cover 508 is affixed directly to the base 502 along its periphery, except to the extent that cover 508 is shown as being partially removed from the base 502 at housing 504, in order to maintain segregation of the suture portions 556 and 558 with a single cover, cover 508 is also affixed to base 502 between the two housings. The attachment of cover 508 to base 502 between the housings 504 and 514 is discontinuous in order to allow a portion of the suture 550 to extend between the housings 504 and 514. Thus, the portion of suture 550, including transitional segment 560, rests in passage 524 between the housings 504 and 514, being bounded at housings 504 by housing aperture 510 and at housing 514 by housing aperture 520.

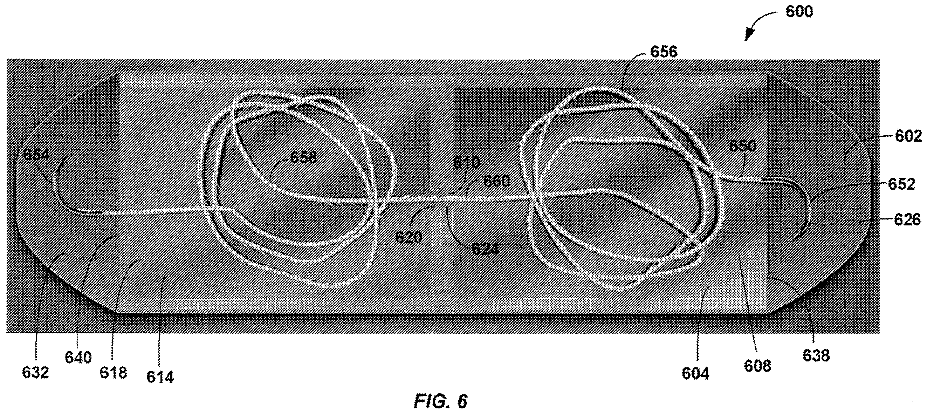

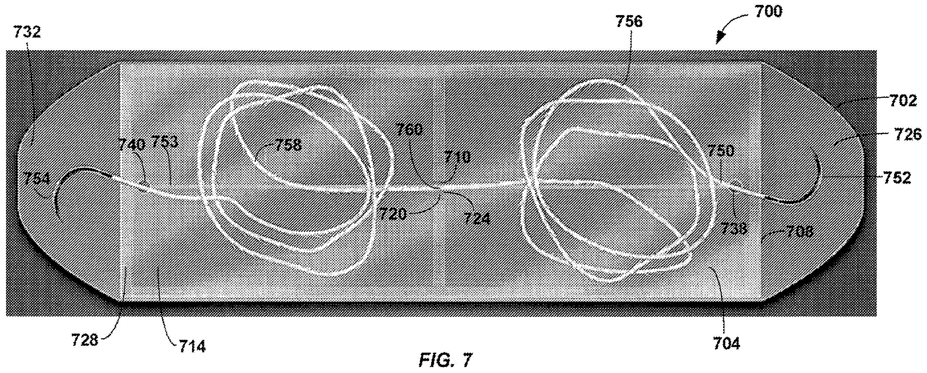

Referring now to FIGS. 6 and 7, additional examples of two-housing suture packages with single covers are depicted. FIG. 6 shows a suture package 600 releasably securing suture 650. Suture package 600 includes base 602 on which are formed housings 604 and 614 sharing a single cover 608. Housing 604 houses a portion of suture 650 having a plurality of retainers 656 oriented in one direction as well as needle 652 attached to an end of the suture 650. Housing 614 houses a portion of suture 650 having a plurality of retainers 658 oriented in a substantially opposite direction to retainers 656 as well as needle 654 attached to an end of the suture 650. Cover 608 is affixed directly to the base 602 along its periphery, but is left open at edges 638 (at housing 604) and 640 (at housing 614) to allow retrieval of portions 656 and 658, respectively, of suture 650. However, open ends 638 and 640 of cover 608 are associated with extensions of base 602 that act as flaps 626 and 632, respectively, that can be folded over ends 638 and 640 in order to close them off. As such, although suture needles 652 and 654 are shown in FIG. 6 as resting on flaps 626 and 632, respectively, it is to be understood that the needles can be tucked into housings 604 and 614 when flaps 626 and 632 are closed over cover 608. Cover 608 is also affixed to base 602 between the two housings, and this attachment is discontinuous in order to allow a portion of the suture 650 to extend between the housings 604 and 614. Thus, the portion of suture 650, including transitional segment 660, rests in passage 624 between the housings 604 and 614, being bounded at housings 604 by housing aperture 610 and at housing 608 by housing aperture 620.

The exemplary suture package 700 of FIG. 7 has a similar cover and flap combination as that of FIG. 6, but with a difference. Suture package 700 includes base 702 on which are formed housings 704 and 714 sharing a single cover 708. Housing 704 houses a portion of suture 750 having a plurality of retainers 756 oriented in one direction as well as needle 752 attached to an end of the suture 750. Housing 714 houses a portion of suture 750 having a plurality of retainers 758 oriented in a substantially opposite direction to retainers 756 as well as needle 754 attached to an end of the suture 750. Cover 708 is affixed directly to the base 702 all the way along its periphery 728. Unlike the open ends 638 and 640 of cover 608 (shown in FIG. 6), however, cover 708 of suture package 700 is provided with apertures 738 and 740 in housings 704 and 714, respectively, for removal of the suture. Moreover, apertures 738 and 740 can act as needle parks to secure needles 752 and 754 for easily grasping the needles. As well, portions of base 702 act as flaps 726 and 732, respectively, that can be folded over housings 704 and 714, respectively, and can close off apertures 738 and 740. Suture needles 752 and 754 can either be tucked into housings 704 and 714 when flaps 726 and 732 are closed over cover 708 and then threaded through apertures 738 and 740 when the flaps 726 and 732 are opened, or else they can rest between cover 708 and flaps 726 and 732, respectively, when the flaps are closed; this latter arrangement could be accomplished by folding over suture 750 at or near apertures 738 and 740. Cover 708 is also affixed to base 702 between the two housings, and this attachment is discontinuous in order to allow a portion of the suture 750 to extend between the housings 704 and 714, such that transitional segment 760 can rest in passage 724, which passage 724 is bounded at housing 704 by housing aperture 710 and at housing 714 by housing aperture 720. Finally, cover 708 is provided with a perforated "tear line" 753 running between apertures 738 and 740 to facilitate release of suture 750 from the package 700.

It is to be understood that the arrangement of the segregated compartments in Figures 3 through 7 are depicted side-by-side only for convenience. As long as the two compartments are separated, their orientation relative to each other is irrelevant; for example, the compartments can be stacked on top of each other or stacked in a staggered configuration; separated by horizontal divider or vertical divider to name just a few possibilities. It is also to be understood that although Figures 3 through 7 depict the suture material simply housed in the two segregated compartments, the suture material can also be wound, spooled, guided or otherwise organized in one or both compartments without deviating from the scope of this disclosure; examples of this particular embodiment are presented in subsequent figures.

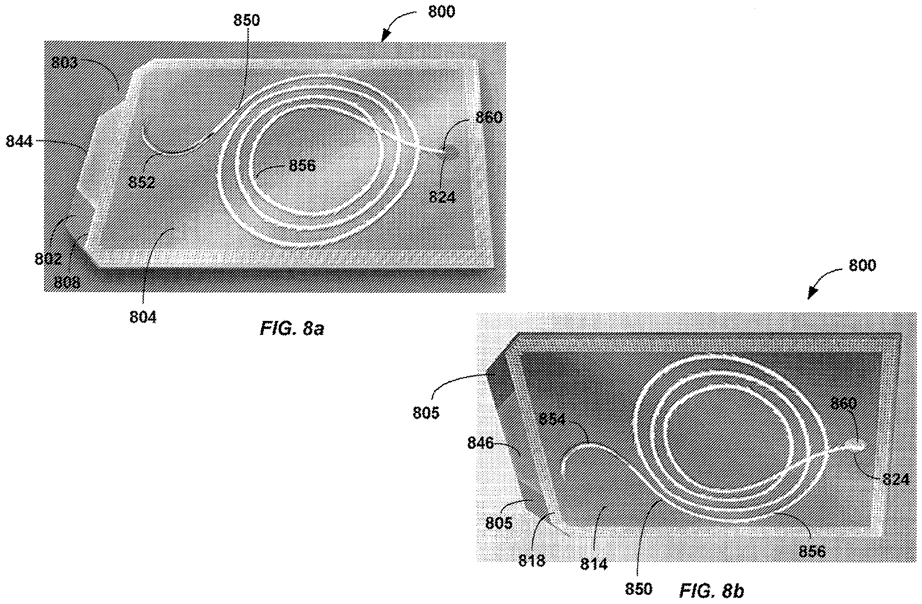

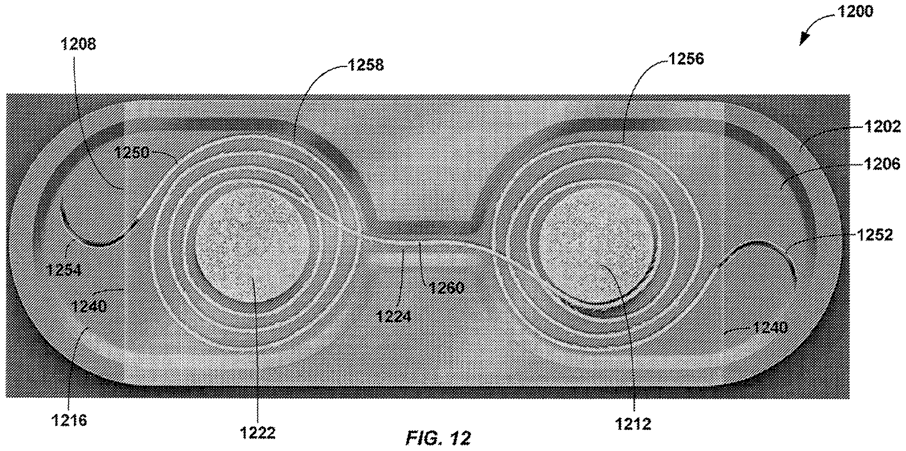

It is also possible to have the segregated suture holders (which, in the examples described in this section, are housings or compartments) on alternate surfaces of the base. Referring now to FIGS. 8a and 8b, suture package 800 releasably secures suture 850. Suture package 800 includes base 802 having surfaces 803 and 805 on which are disposed housings 804 and 814, respectively. Housing 804 includes cover 808 and houses a portion of suture 850 having a plurality of retainers 856 oriented in one direction, while housing 814 includes cover 818 and houses a portion of suture 850 having a plurality of retainers 858 oriented in a substantially opposite direction to retainers 856. Covers 808 and 818 are affixed directly to the base around the covers' respective edges, and so suture needles 852 and 854 also remain inside housings 804 and 814, respectively. Covers 808 and 818 are provided with tabs 844 and 846, respectively, that can be grasped to remove covers 808 and 818 in whole or in part. As housings 804 and 814 are on different surfaces of the base 802, base 802 is provided with an aperture 824 communicating the two housings 804 and 814. A portion of suture 850, including transitional segment 860, rests between the two housings, in connecting aperture 824. Although the needles are depicted in FIGS. 8a and 8b as being contained with their respective compartments, one or both of them can be placed partially or completely outside of the compartment (i.e. emerging from their compartment through the sides or through an aperture in covers 808 and/or 818) to facilitate grasping of the needle with a needle driver. It should also be obvious to one of skill in the art that although FIGS. 8a and 8b depict the suture material simply housed in the two segregated compartments, the suture material can also be wound, spooled, guided or otherwise organized in one or both compartments without deviating from the scope of this disclosure (an example of which is presented in Figure 12).

If it is desired to seal off the segregated portions (for example, in order to maintain sterility of those portions), the cover may be affixed to the base after the suture is arranged in the desired segregated configuration on the base, such that the only points that the base and covers are not in contact with one another are the points at which the middle portion of the suture extends out of the respective housings and, in some cases as discussed herein, at which the deployment ends of the suture come out of the respective housings. A cover may be attached to a base by methods as varied as applying adhesive to stick each cover to the base, sewing them together, heat sealing them together, providing a hinge along one or a portion of an edge of a cover, and so forth. The attachment may be continuous along the cover and base (except of course at the apertures through which a suture is to pass through), or discontinuous, with as many points of attachment as are desired or as few as are necessary to keep the cover on the base. As the cover and base may be made of any suitable materials, the minimum number of attachment points would depend on factors understood by those skilled in the art, such as the characteristics of the materials selected for the covers and base (including, without limitation, characteristics such as rigidity and resilience), the strength of each attachment point, the weight and/or bulk of the sutures being housed, and so forth. Likewise, the choice of materials and attachment method would depend on how the suture is to be removed from the housing; for example, if the cover is to be peeled off, then the attachment of the cover to the base might desirably be effected with a sufficiently weak adhesive, or with sufficiently few points of heat-sealing. Alternatively, the choice of attachment may be less important if the cover or base is to be torn or cut in order to free a suture, if the cover or base is to be torn, then at least a sufficient portion of the cover or base should consist of a tearable material (such as paper), while if the cover or base is to be cut then at least a sufficient portion of the cover or base should consist of a material that can be cut through with a sharp object. Likewise, the choice of attachment is less important than the choice of housing material if some part of the housing is to be snapped off or otherwise broken to release a suture.

### B. Guides

While the foregoing examples describe surrounding portions of a suture in order to segregate them, a suture holder may as well or instead be provided with a suture guide to segregate a portion of the suture. Guides may include without limitation spools (stationary or rotatable), reels, and winding elements around which the suture may be wound, arches or bridges under which the suture may pass in a coil, eccentric guide elements around which a suture can be wound (such that the guide elements act something like foci in an ellipse), one or more perforations in the base through which a suture may pass (thus using the base itself as a guide element; see, for example, U.S. Patent Application. No. 10/420,993,

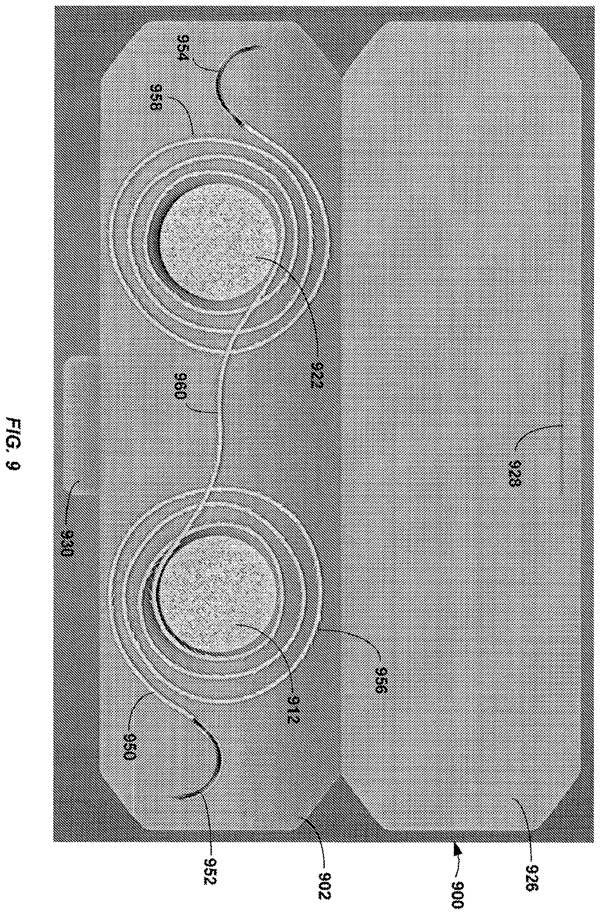

With reference to FIG. 9, suture package 900, shown in an open position, is provided with spools 912 and 922 on base 902, and releasably secures suture 950. Suture portion 956 having a plurality of retainers oriented in one direction is coiled around spool 912 and needle 952 is disposed proximally thereto. Similarly, suture portion 958 having a plurality of retainers oriented in another direction to that of portion 956 is coiled around spool 922 and needle 954 is disposed proximally thereto. A portion of suture 950 bearing the transitional segment 960 extends between spools 912 and 922. To prevent suture 950 from disengaging from the package 900 before the surgeon intends to remove the suture 950, package 900 is further provided with a cover flap 926 that can fold over spools 912 and 922 and can be held in such a closed position by mating tab 930 with slot 928. Of course, it is to be understood that the tab/slot combination is only one of many fastening means that would be obvious to one skilled in the art. Cover flap 926 can also be fashioned such that it covers only spool 912 or 922 (but not both) to help facilitate deploying the two halves of the suture sequentially during clinical use (the "uncovered" spool being deployed first and the "covered" spool deployed second after removal of the cover).

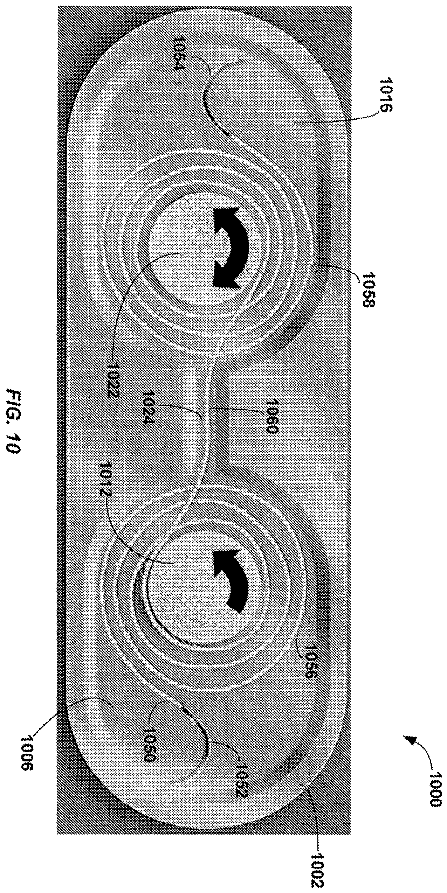

FIG. 10 depicts an alternate spool configuration Suture package 1000 is provided with spools 1012 and 1022 disposed in base 1002, and releasably secures suture 1050. Suture portion 1056 having a plurality of retainers oriented in one direction is coiled around spool 1012 and needle 1052 is disposed proximally thereto. Similarly, suture portion 1058 having a plurality of retainers oriented in another direction to that of portion 1056 is coiled around spool 1022 and needle 1054 is disposed proximally thereto. Spools 1012 and 1022 are rotatable, as indicated by the rotational arrows; while both spools can rotate in a direction appropriate to dispense at least a portion of the suture 1050 mounted on that spool, spool 1022 can rotate in the opposite direction as well, so as to allow the suture 1050 to be drawn onto spool 1012. Suture 1050 is wound in one direction around spool 1012 and wound in the opposite direction around spool 1022 in order to facilitate removal by rotation of the respective spools. Of course, it is to be understood that both spools can be configured to rotate in one or both directions. Other exemplary rotatable spools are described in U.S. Patent Nos. 5,918,733 and 6,254,616. Base 1002 is recessed to provide wells 1006 and 1016 associated with spools 1012 and 1022, respectively; such that each spool and well combination comprises a suture holder. The depth of the wells provide a greater volume for portions 1056 and 1058 to occupy, thereby lessening the probability that the suture 1050 will retain a memory of its coiled configuration when it is removed from package 1000. A portion of suture 1050 bearing the transitional segment 1060 extends between spools 1012 and 1022, through a recessed portion of base 1002 that forms passage 1024 joining the two suture holders. Suture package 1000 can be further provided with a cover (such as the covers described with reference to FIGS. 3 to 8, inclusive), or with a flap like the one described in relation to FIG. 9, or other covering elements.

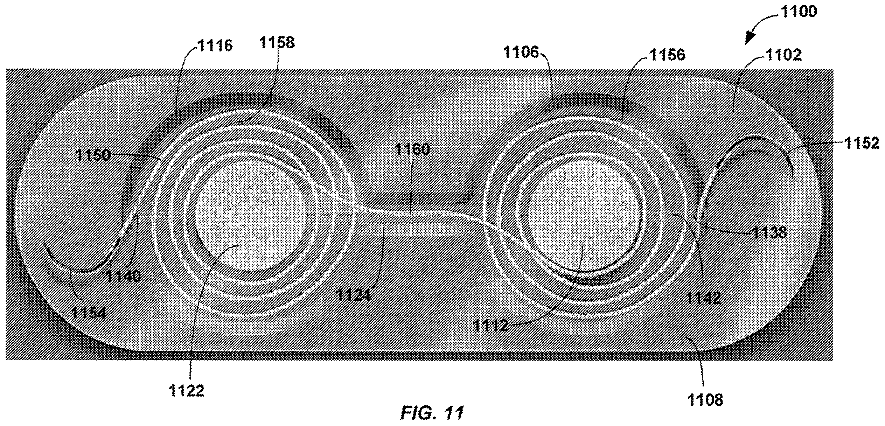

FIGS. 11 and 12 depict suture packages including at least partially covered spool arrangements in which the ends (whether armed or not) of the packaged suture are exposed to facilitate grasping of the ends. As in suture package 1000 of FIG. 10, the suture package 1100 FIG. 11 includes base 1102 with recessed wells 1106 and 1116 provided with spools 1112 and 1122, respectively, and releasably secures suture 1150. Suture portion 1156, having a plurality of retainers oriented in one direction, is coiled around spool 1112 and needle 1152 is disposed proximally thereto. Similarly, suture portion 1158, having a plurality of retainers oriented in another direction to that of portion 1156, is coiled around spool 1122 and needle 1154 is disposed proximally thereto. A portion of suture 1150 bearing the transitional segment 1160 extends between spools 1112 and 1122, through a recessed portion of base 1102 that forms passage 1124 joining the two suture holders. Suture package 1100 is further provided with cover 1108 that engages base 1102 along at least the perimeter of the non-recessed portion of base 1102, to prevent suture 1150 from falling out of the package 1100. Cover 1108 is provided with apertures 1138 and 1140 through which are exposed needles 1152 and 1154, respectively, and through which suture 1150 may be drawn out of package 1100 by pulling on such needles. To facilitate complete removal of suture 1100, cover 1108 can be removed by lifting it away the base along opening 1142 extending between apertures 1138 and 1140.

Referring now to FIG. 12, suture package 1200 releasably secures suture 1250 and is similarly provided with base 1202 having recessed wells 1206 and 1216 in which are disposed spools 1212 and 1222, respectively. Suture portion 1256, having a plurality of retainers oriented in one direction, is coiled around spool 1212 and needle 1252 is disposed proximally thereto. Similarly, suture portion 1258, having a plurality of retainers oriented in another direction to that of portion 1256, is coiled around spool 1222 and needle 1254 is disposed proximally thereto. A portion of suture 1250 bearing the transitional segment 1260 extends between spools 1212 and 1222, through a recessed portion of base 1202 that forms passage 1224 joining the two suture holders. Suture package 1200 is further provided with cover 1208 that engages base 1202 along part of the perimeter of the non-recessed portion of base 1202, but unlike cover 1108 of the exemplary package of FIG. 11, cover 1208 does not extend to the ends of the perimeter of base 1202. Rather, cover 1108 has a pair of edges 1240 that provide access to recessed wells 1206 and 1216 for removal of needles 1152 and 1154, respectively. If desired, cover 1208 can be removed by grasping it at either of edges 1240 and lifting it away base 1202.

It is to be understood that the segregated compartments depicted in Figures 10 through 12, inclusive, are depicted side-by-side only for convenience. As long as the two housings are separated, their orientation relative to each other is irrelevant; for example, without limitation, the housings can be stacked on top of each other or arranged in a staggered configuration, separated by horizontal or vertical dividers to name just a few possibilities. Also, it should be noted that the cover flap can also be fashioned such that it covers only one spool (but not both) to help facilitate deployment of the two halves of the suture sequentially during clinical use (the "uncovered" spool being deployed first and the "covered" spool deployed second after removal of the cover).

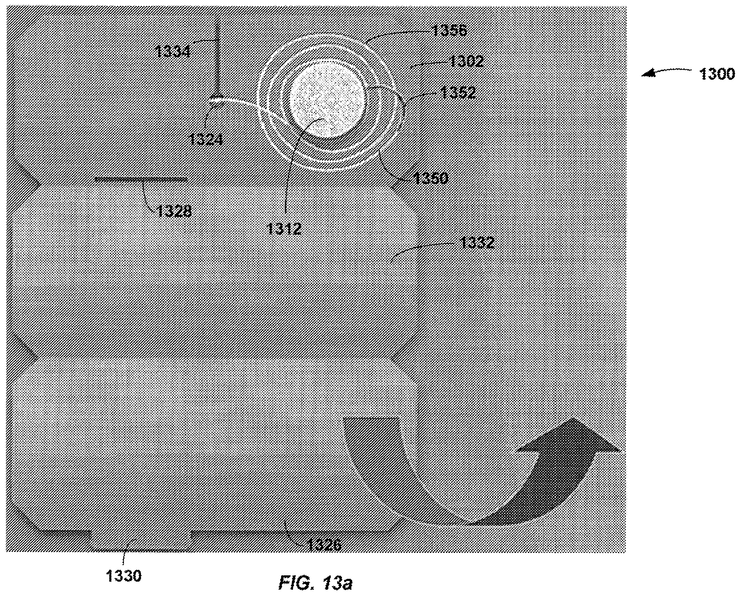

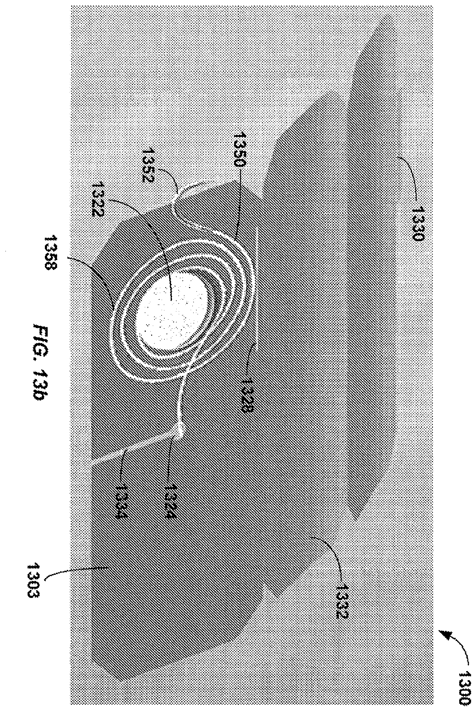

Referring now to FIGS. 13a and 13b, suture holders that have guide elements rather than housings can also be provided on differing base surfaces. Suture package 1300 releasably secures suture 1350. Suture package 1300 includes base surfaces 1302 and 1303 on which are disposed spools 1312 and respectively. A portion of suture 1350 having a plurality of retainers 1356 oriented in one direction is coiled around spool 1312, while a portion of suture 1350 having a plurality of retainers 1358 oriented in a substantially opposite direction to retainers 1356 is coiled around spool 1322. As shown in FIG. 13a, a spool (in this case, spool 1312) can be used as a needle park to secure a needle (in this case, needle 1352). To prevent suture 1350 from disengaging from the spools 1312 and 1322 before the surgeon intends to remove the suture 1350, package 1300 is further provided with a cover to hold suture 1350 in place. Thus, cover segment 1326 can fold over into engagement with spool 1312 while cover segment 1332 engages spool 1322, and both can be held in such a closed position by mating tab 1330 with slot 1328, resulting in a "layered" packaging. (Of course, it is to be understood that the tab/slot combination is only one of many fastening means that would be obvious to one skilled in the art). During deployment, the user may disengage both cover segments from their respective spools, or may wish to first expose spool 1312 by removing cover portion 1326 while still holding cover portion 1332 in engagement with spool 1332. As spools 1312 and 1322 are on different surfaces of the base 1302 and 1303, the base is provided with an aperture 1324 communicating the two spools 1312 and 1322, and a channel 1334 for ease of removal of suture 1350. A portion of suture 1350, including transitional segment between pluralities of retainers 1356 and 1358, rests between the two housings, in connecting aperture 1324.

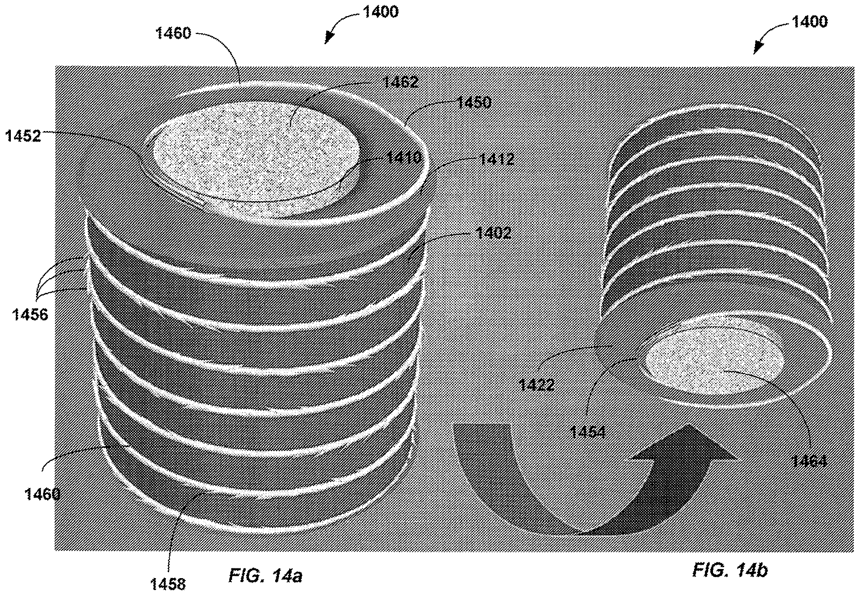

Each plurality of retainers facing in a given direction on a bidirectional suture can be segregated in a suture package by wrapping the suture on a guide assembly and without allowing any overlap or intersection of the retainer pluralities, such that each end of the suture is dispensable from a separate location on the guide assembly. Referring now to FIGS. 14a and 14b, suture package 1400 is a guide assembly having a cylindrical base (that is, a spool) 1402 with caps 1412 and 1422 at each end and needle parks 1462 and 1464 disposed on each cap 1412 and 1422, respectively. Suture 1450 is wrapped around guide assembly 1410, and plurality 1456 of retainers facing in a given direction is in spaced relation to plurality 1458 of retainers facing in an opposing direction (and which is separated from retainer plurality 1456 by transitional segment 1460). The base of the guide assembly may be provided with grooves or channels for each retainer plurality in order to maintain each in segregation from the other, or the suture may be wrapped sufficiently tightly around the guide assembly that the retainer pluralities do not overlap with one another; for example, needles 1452 and 1454 at either end of suture 1450 may be removably engaged in needle parks 1462 and 1464, respectively, in order to achieve such tension.

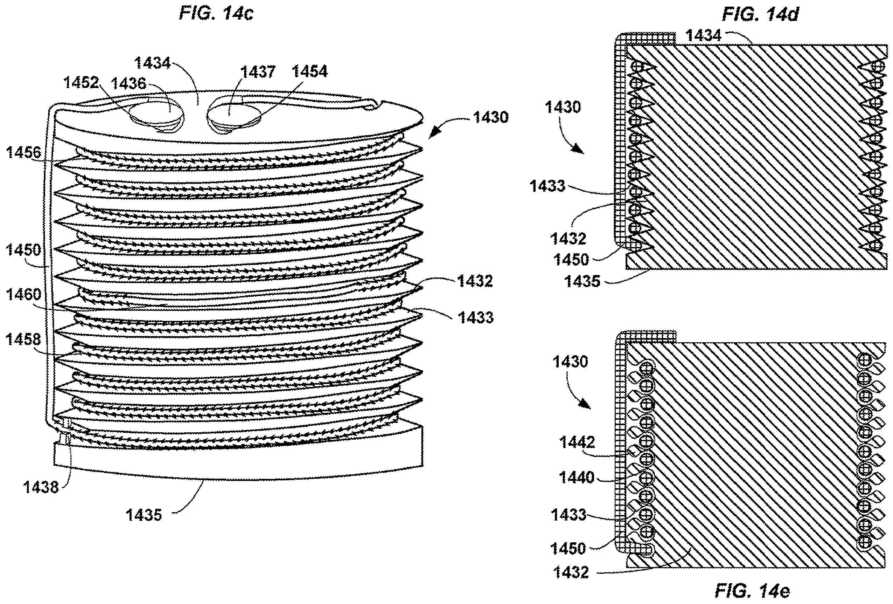

As shown in FIGS. 14c and 14d, suture package 1430 is a guide assembly having a cylindrical base (that is, a spool) 1432 with caps 1434 and 1435 at each end and needle parks 1436 and 1437 disposed on the same cap 1434 so that they can be accessed from a single end of suture package 1430. A helical or spinal groove (channel) 1433 runs around the circumference of cylindrical base 1432 progressing from cap 1434 to cap 1435. Helical groove 1433 is deep enough to contain a suture. Although a v-shaped groove is shown the groove/channel may be rounded square or have an alternative geometry suitable for receiving a suture and maintaining a separation between adjacent turns of suture around cylindrical base 1432. Note that in some embodiments, cylindrical base 1432 may be a hollow cylindrical base such that one or more suture packages 1430 of decreasing diameter may be nested inside another suture package 1430.

As shown in FIG. 14c, suture 1450 is wrapped around guide assembly 1430 within helical groove 1433, and plurality of retainers 1456 facing in a given direction is in spaced relation to plurality of retainers 1458 facing in an opposing direction (and which is separated from retainer plurality 1456 by transitional segment 1460). The suture may be wrapped sufficiently tightly around the guide assembly that the retainer pluralities do not overlap with one another, for example, needles 1452 and 1454 at either end of suture 1450 may be removably engaged in needle parks 1436 and 1437, respectively, in order to achieve such tension. In this embodiment, a releasable clip 1438 holds suture 1450 to cap 1435 to maintain tension in suture 1450 when suture 1450 passes vertically up the side of base 1432 between cap 1435 and cap 1434. Note that as seen in the sectional view of FIG. 14d, helical groove 1433 is sufficiently deep that the vertical portion of suture 1450 is segregated from the portion of suture 1450 within helical groove 1433. Note that in use, needle 1452 must be released first and then suture 1450 may be released from clip 1438 before being unwound from the spool. It is to be understood that in this particular embodiment that instead of a release clip that friction engagement structures can be used to retain the suture in place in grooves. Such friction engagement structures are depicted in the other embodiments by way of example only.

Fig. 14e shows an alternative configuration for helical grove 1433 of suture package 1430. As shown in Fig. 14e, the helical groove 1433 is wider closer to the center of the spool than towards the perimeter. Helical groove 1433 thus comprises a helical pocket 1440 into which suture 1450 may be fit. The adjacent turns of helical wall 1442 of helical groove 1433 are slightly closer together than the diameter of the suture 1450 and thus serve to keep suture 1450 in the pocket even when the tension in suture 1450 is released. Wall 1442 is flexible enough that it can be pushed apart with a slight force to admit and release suture 1450 as it is being wound on or off of the spool. This configuration of helical groove 1433 forms what is essentially a continuous helical clip which lightly holds suture 1450 to the surface of cylindrical base 1432. Other configurations of groove and clips may be similarly used to hold suture 1450 in place. For example, releasable clips (like clip 1438) may be provided intermittently along helical groove 1433. Alternatively a releasable adhesive or gel may be provided continuously or intermittently along helical grove to retain suture 1450 in the absence of tension.

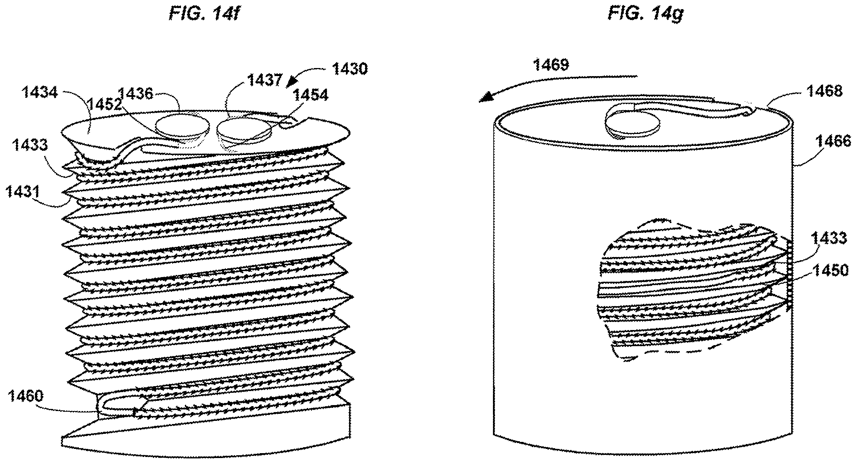

FIG. 14f shows a variation of suture package 1430. The guide assembly has a cylindrical base (that is, a spool) 1432 and a pair of helical or spinal grooves (channel) 1431 and 1433 which run around the circumference of cylindrical base 1432 in a double helix relationship. The double helix formed allows for both arms of suture 1450 to be wound around the cylindrical housing while being segregated by the walls of the grooves 1433. Needles 1452 and 1454 at either end of suture 1450 may be removably engaged in needle parks 1436 and 1437, on the same cap 1434 of the spool. At transition region 1460, suture 1450 crosses over between groove 1431 and groove 1433 and doubles back on itself towards the upper cap 1434.

FIG. 14g shows another variation of suture package 1430. The guide assembly has a cylindrical base (that is, a spool) 1432 and helical groove (channel) 1433 which runs around the circumference of cylindrical base 1432. Helical groove 1433 is deep enough to contain a suture. In the embodiment of FIG. 14f the spool is provided with a slotted cover 1466 which fits over the spool, holding suture 1450 within groove 1433. Suture 1450 can be removed through slot 1468. Cover 1466 rotates around the spool as shown by arrow 1469 so that suture 1450 may be unwound from groove 1433 through slot 1468. The slotted cover 1466 may also be utilized with a suture package having a double helix arrangement of grooves as shown in FIG. 14f. In alternative embodiments, cover 1466 may be a fixed cover without a slot. In order to remove suture 1450, the cover may be progressively slid off suture package 1430. Alternatively cover 1466 may be made of a material that can be split by the suture as the suture 1450 is unwound Cover 1460 may, for example be perforated along the groove 1433 or grooves 1431, 1433 so that suture 1450 may be pulled through the cover 1466 as it is unwound from the spool.

### C. Combinations of Housing and Guide

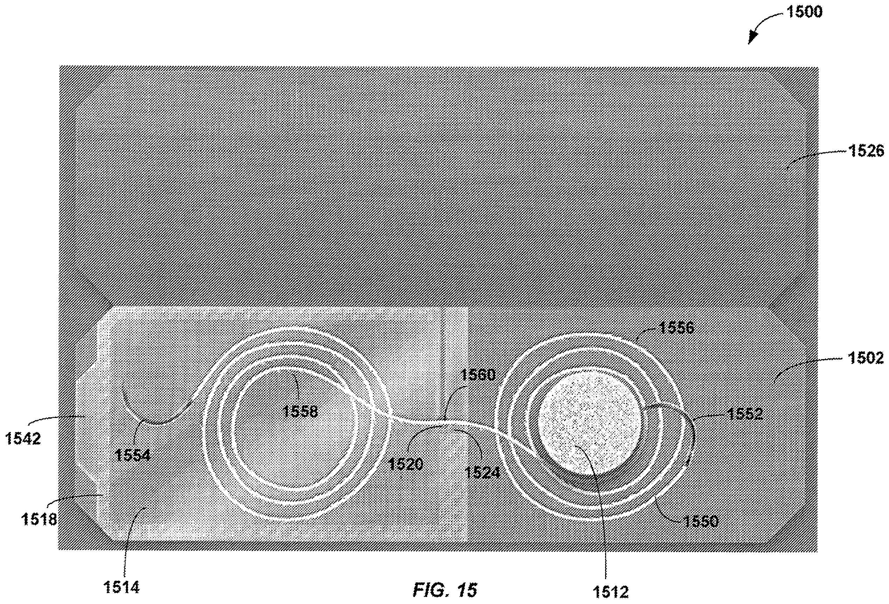

As already discussed in examples herein, suture packages can also be provided with any combination of housing and guide disclosed herein. Referring for example to FIG. 15, suture package 1500 releasably secures suture 1550. Suture package 1500 includes base 1502 on which are disposed spool 1512 and housing 1514. Suture portion 1556 having a plurality of retainers oriented in one direction is coiled around spool 1512 and needle 1552 is engaged in spool 1512, such that spool 1512 also functions as a needle park. Housing 1514 includes cover 1518 and houses a portion of suture 1550 having a plurality of retainers 1558 oriented in a substantially opposite direction to retainers 1556. Cover 1518 is affixed directly to the base most of the way around the cover's edges, so that suture needle 1554 remains inside housing 1514. Cover 1518 is also provided with tab 1542 that can be grasped to remove cover 1518 in whole or in part. Housing 1514 is provided with an aperture 1520 through which suture 1550 can extend to spool 1512. A portion of suture 1550 including transitional segment 1560 rests between the two housings, in connecting passage 1524 which is bounded on one end by aperture 1520. Package 1500 is further provided with a flap 1526 that can be folded over to cover spool 1512 and housing 1514.

### DEPLOYMENT OF SUTURES FROM SUTURE PACKAGES

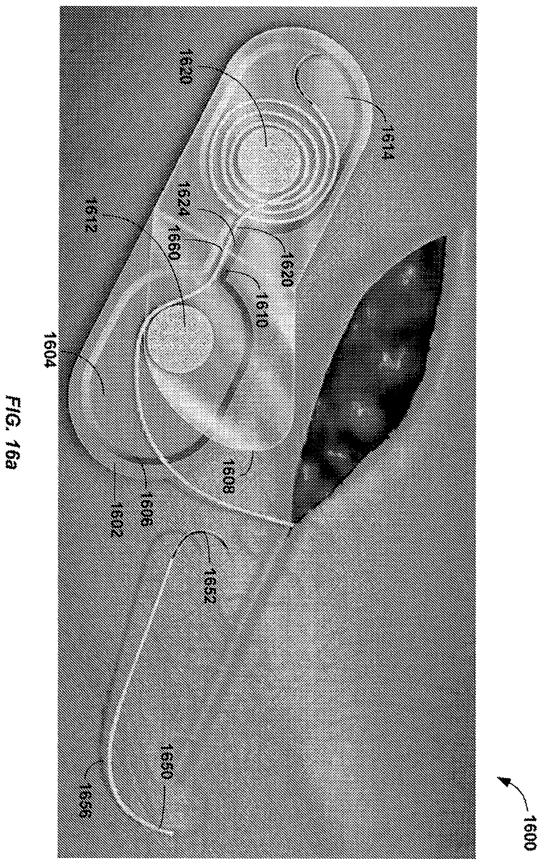

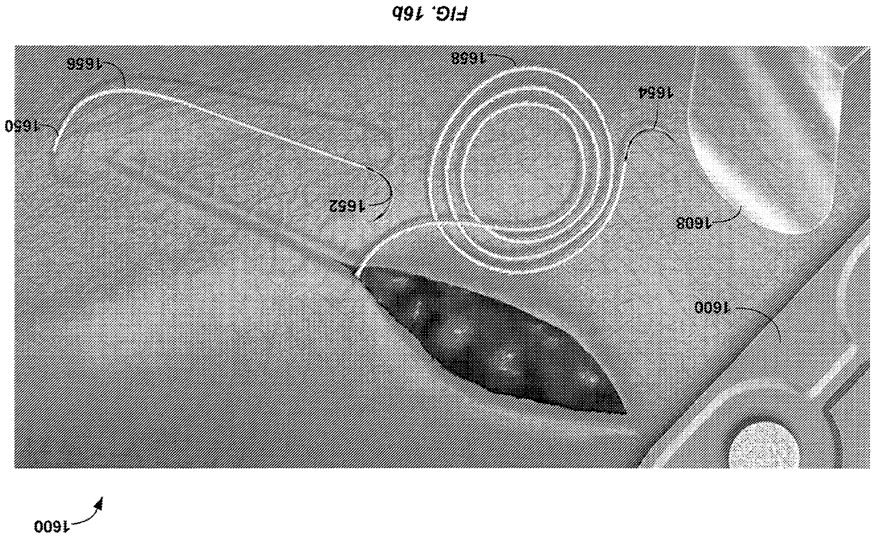

A use of a bidirectional serf-retaining suture 1650 dispensed from a double spool and housing suture package 1600 is depicted in FIGS. 16a and 16b. With reference to FIG. 16a, suture 1650 is wound around spools 1612 and 1620 of first housing 1604 and second housing 1614, respectively. A portion of the suture 1650 extends between the housings 1604 and 1614. Thus, the portion of suture 1650, including transitional segment 1660, rests in passage 1624 between the housings 1604 and 1614, being bounded at housings 1604 by housing aperture 1610 and at housing 1614 by housing aperture 1620. Cover 1608 is lifted from base 1602 at first housing 1604 and suture 1650 is deployed using needle 1652 through wound edges from about the central portion of the wound to one end of the wound. First plurality of retainers 1656, disposed proximal to and facing away from needle 1652, is pulled through tissue at the wound edges in the deployment direction. As illustrated in FIG. 16a, upon subcuticularly connecting wound edges with stitches running from the center to the end of the wound, suture 1650 is pulled in the deployment direction to draw the wound edges together. Referring now to FIG. 16b, cover 1608 is removed completely from base 1602 and the remainder of suture 1650 is removed therefrom. The remainder of suture 1650, bearing the second plurality of retainers disposed proximal to and facing away from needle 1654, may be used to repeat the foregoing process in the opposing direction for the rest of the wound. When, on the second half of the wound closure surgery, suture 1650 is drawn through the tissue to approximate the wound edges on the open remainder of the wound, the act of pulling the suture 1650 in the second deployment direction (that is, towards the second end of the wound) comprises the necessary affixation force for the first plurality of retainers 1656, thus causing first plurality of retainers 1656 to engage the tissue. Conversely, once suture 1650 is pulled sufficiently tightly to close the second half of the wound, the engagement force of the tissue exerted against the first plurality of retainers 1656 affixes the second plurality of retainers 1658.

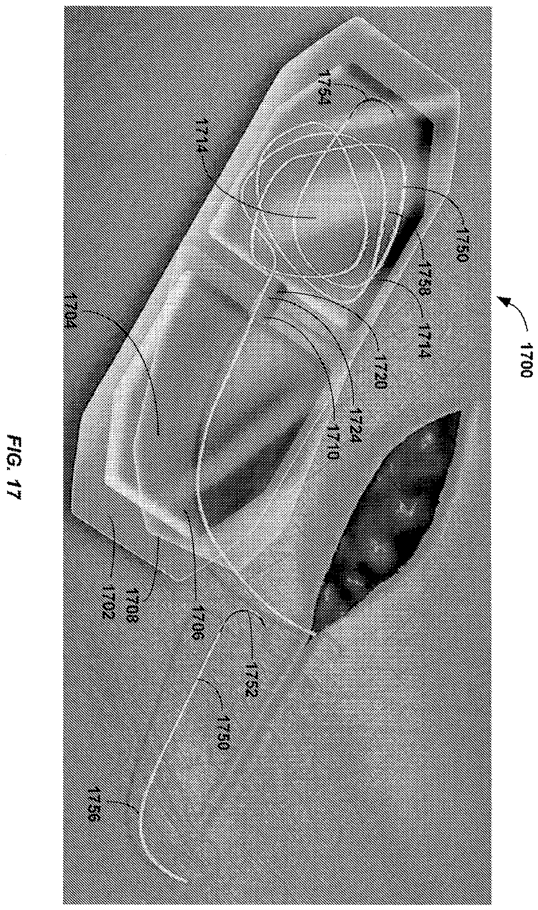

Similarly, FIG. 17 depicts the deployment of a bidirectional self-retaining suture 1750 from a double-housing suture package 1700. As shown in FIG. 17, a first portion of suture 1750 having a first plurality of retainers 1756 oriented away from first needle 1752 is deployed into the wound through cover 1708 that is sufficiently open over first housing 1704 to provide an exit for suture 1750. A portion of the suture 1750 extends between the housings 1704 and 1714. Thus, the portion of suture 1750, including transitional segment 1760, rests in passage 1724 between the housings 1704 and 1714, being bounded at housing 1704 by housing aperture 1710 and at housing 1714 by housing aperture 1720. As can be seen in FIG. 17, a second portion of suture 1750 having a second plurality of retainers 1758 oriented away from second needle 1754 remains for the most part in suture package 1700; the portion bearing the second plurality of retainers 1758 can be seen to be drawn through aperture 1720 of second housing 1714 into first housing 1704 and out of the package 1700. As there is no spool (stationary or otherwise) in either housing 1704 or 1714, cover 1708 can remain in place over second housing 1714 without preventing the complete removal of suture 1750 from housing 1714. Thus, in dispensing suture 1750 from suture package 1700, the suture 1750 may be drawn out of the package 1700 from either one of the holders 1704 or 1714, such that when the portion that had previously been maintained by that holder is being drawn out, the portion of the suture 1750 in the other holder is eventually pulled through passage 1724 and out of the holder from which the first portion of suture 1750 had been dispensed. In this fashion it is necessary to open only one of the holders in order to fully release suture 1750 for deployment into tissue. It should be noted that at the operator's discretion, cover 1708 can also be completely removed to expose the second housing 1714 (typically after the first half of the wound has been closed) should this be deemed to be more convenient.

As described in the two preceding examples, the segment of a bidirectional suture having retainers in a single direction can be drawn progressively from a sterile package without affecting the segment of the suture having retainers in a substantially opposing configuration. The second segment can be accessed in a similar manner when required.

As well, it should be noted that sewing with bidirectional sutures typically involves pulling the suture through both sides of the wound at (or near) the middle of the wound to be closed until the retainers of the opposite configuration "grab" the tissue. While the foregoing examples disclose having only retainers of a single orientation in each suture holder, the use of the suture package could further facilitate clinical deployment by including a length of suture (several centimetres (inches)) of the opposite retainer configuration (i.e the region closest to the transition segment) in the suture housing that contains the part of the suture that will be deployed into the wound first. This arrangement may allow a surgeon to have enough "slack" to completely close the first half of the wound using only suture deployed from the first housing of the package; the remaining suture from the second housing could then be released to close the second half of the wound. While this would slightly increase the chances of the different barb configurations "catching" (although the incidence would still be much smaller than if the entire suture were contained in one housing), this problem could be alleviated by using a spooling (or winding) mechanism of suture storage that prevented the barbs from coming into direct contact (as described above). In this regard, it should be noted that while the suture packages disclosed herein may optimally prevent or reduce catching by maintaining only retainers of a single orientation in each suture holder, this is not the only way to use these packages with bidirectional sutures, and indeed while a plurality of retainers of a single orientation may be maintained in each holder, it is possible to have retainers of that orientation in the other holder or outside the holders altogether.

### MANUFACTURE AND BIOLOGICAL AGENTS

While manufacturing has been disclosed in greater detail in the foregoing examples, it is worth reiterating that these suture packages may be manufactured from any suitable material and, depending on the configuration of the selected suture holders, may have sutures packaged into them during the manufacturing process or after the packages are manufactured. Some of these examples describe suture packages in which sutures are fully sealed and therefore can be maintained in sterility, but it is to be understood that any of the packages disclosed herein can be further enclosed in sealed containers which would facilitate maintaining sterility. Furthermore, these suture packages may be provided with compositions to facilitate dispensing of the sutures, to inhibit growth of bacteria, fungi, or other contaminants, or to be transferred on to the sutures themselves (such as compounds to promote healing and prevent undesirable effects such as scar formation, infection, pain, and so forth). This can be accomplished in a variety of manners, including for example: (a) by directly affixing to the desired surface(s) of the package a formulation (e.g., by either spraying the suture with a polymer/drug film, or by dipping the suture into a polymer/drug solution), (b) by coating the desired surface(s) of package with a substance such as a hydrogel which will in turn absorb the composition, or (c) manufacturing the package itself with a composition. Such compositions may include without limitation anti-proliferative agents, anti-angiogenic agents, anti-infective agents, fibrosis-inducing (or "pro-healing") agents, anti-scarring (or anti-adhesion) agents, lubricious agents, echogenic agents, radio-opaque agents (and/or agents that enhance visualization under X-ray, CT, MRI and/or PET scanning), antiinflammatory agents, cell cycle inhibitors, radioactive isotopes, analgesics, anaesthetics and anti-microtubule agents. For example, without limitation, the surfaces of a package that may contact the suture may have a composition applied thereto prior to the packaging of the suture, so that when the suture is dispensed from the package the composition may contact and adhere to the suture. For suture packages having one or more housings, the interior surfaces of the housings may be so provided with the desired composition, while for those having guides the guides may be so provided with the desired composition. Similarly, any part of the package with which the suture comes into contact may be provided with the composition, such as surfaces of the base or, if a suture passes through the base, then the passage in the base. Alternatively, the suture package may include a composition-dispensing container through which the suture may be passed prior to deployment into tissue. The purpose of the suture may also determine the sort of composition that is applied to the suture package; for example, anti-proliferative coatings (drugs or radioactive isotopes) may be desired for packages for sutures used in closing tumour excision sites, packages containing self-retaining sutures for use in tissue repositioning procedures may be provided with fibrosing (agents that promote wound healing)coatings, and those having anti-scarring or anti-infective coatings may be used for wound closure sutures. Coatings may also include a plurality of compositions either together or on different portions of the suture, where the multiple compositions can be selected either for different purposes (such as combinations of analgesics, anti-infective and anti-scarring agents).

Although the present invention has been shown and described in detail with regard to only a few exemplary embodiments of the invention, it should be understood by those skilled in the art that it is not intended to limit the invention to the specific embodiments disclosed. Various modifications, omissions, and additions may be made to the disclosed embodiments without materially departing from the novel teachings and advantages of the invention, particularly in light of the foregoing teachings. Accordingly, it is intended to cover all such modifications, omissions, and additions as may be included within the scope of the invention as defined by the following claims.

## Claims

1. A suture package (1430) adapted to hold at least one suture with first and second segments, and with a first needle extending from the end of the first segment and a second needle extending from the end of the second segment, the suture package comprising:
a cylindrical base (1432);
said cylindrical base including a first end (1434) and a second end (1435);
a first needle park (1436) positioned on one of said first end and said second end, and a second needle park (1437) positioned on one of said first end and said second end;
said cylindrical base adapted to have the suture wrapped about said cylindrical base;
said first needle park adapted to retain the first needle; and
said second needle park adapted to retain the second needle,
**characterised in that** said cylindrical base includes at least one helical channel (1433) that communicates between said first and said second end and is deployed about said cylindrical base,
wherein said channel and said first and second needle park are together adapted to retain said suture such that, when packaged, said suture is wrapped around said cylindrical base within said channel, and said first needle is removably retained in said first needle park and said second needle is removably retained in said second needle park to provide tension,
or wherein releasable clips are provided along said helical channel to retain said suture,
or wherein a releasable adhesive or gel is provided continuously or intermittently along said helical channel to retain said suture,
such that, when packaged, said first segment of said suture is segregated from said second segment of said suture.

2. The package (1430) of claim 1, wherein said at least one channel (1433) is a v-shaped groove.

3. The package (1430) of claim 1, wherein said cylindrical base (1432) has a hollow interior such that one or more suture packages (1430) of decreasing diameter may be nested inside another suture package.

4. The package (1430) of claim 1, wherein said first needle park (1436) is disposed on said first end (1434), and said second needle park is disposed on said second end (1435).

5. The package (1430) of claim 1, wherein said cylindrical base (1432) comprises two helical channels (1431, 1433) arranged in a double helix relationship.

6. The package (1430) of claim 1, further comprising a cover (1466) fitting over the cylindrical base (1432) and covering the outer surface of said cylindrical base.

7. The package (1430) of claim 6, wherein said cover (1466) can be slid off of said cylindrical base.

8. The package (1430) of claim 6, wherein said cover (1466) is slotted and rotatable about said cylindrical base (1432) so as to allow removal of said suture through the slotted portion of said cover as said suture is unwound.

9. The package (1430) of claim 6, wherein said cover (1466) is capable of being split by said suture as said suture is unwound.

## Patentansprüche

1. Nahtmaterialpackung (1430), die geeignet ist, mindestens einen Nahtmaterialfaden mit einem ersten und einem zweiten Segment und mit einer ersten Nadel, die sich vom Ende des ersten Segments erstreckt, und einer zweiten Nadel, die sich vom Ende des zweiten Segments erstreckt, zu enthalten, wobei die Nahtmaterialpackung Folgendes umfasst:
eine zylindrische Basis (1432),
wobei die zylindrische Basis ein erstes Ende (1434) und ein zweites Ende (1435) aufweist,
einen ersten Nadelpark (1436), der auf dem ersten Ende oder dem zweiten Ende positioniert ist, und
einen zweiten Nadelpark (1437), der auf dem ersten Ende oder dem zweiten Ende positioniert ist,
wobei die zylindrische Basis dazu geeignet ist,
dass der Nahtmaterialfaden um die zylindrische Basis herumgewickelt ist,
wobei der erste Nadelpark geeignet ist, die erste Nadel zu halten, und
der zweite Nadelpark geeignet ist, die zweite Nadel zu halten,
**dadurch gekennzeichnet, dass** die zylindrische Basis mindestens einen spiralförmigen Kanal (1433) aufweist, der zwischen dem ersten und dem zweiten Ende kommuniziert und um die zylindrische Basis ausgebracht ist,
wobei der Kanal und der erste und der zweite Nadelpark zusammen geeignet sind, den Nahtmaterialfaden zu halten, so dass der Nahtmaterialfaden, wenn er verpackt ist, um die zylindrische Basis in dem Kanal herum gewickelt ist, und die erste Nadel entfernbar in dem ersten Nadelpark gehalten ist und die zweite Nadel entfernbar in dem zweiten Nadelpark gehalten ist, um für Spannung zu sorgen,
oder wobei freigebbare Clips entlang dem spiralförmigen Kanal zum Halten des Nahtmaterialfadens vorgesehen sind,
oder wobei ein freigebbarer Klebstoff oder ein freigebbares Gel durchgehend oder intermittierend entlang dem spiralförmigen Kanal zum Halten des Nahtmaterialfadens vorgesehen ist,
so dass das erste Segment des Nahtmaterialfadens, wenn er verpackt ist, von dem zweiten Segment des Nahtmaterialfadens getrennt ist.

2. Packung (1430) nach Anspruch 1, wobei der mindestens eine Kanal (1433) eine v-förmige Nut ist.

3. Packung (1430) nach Anspruch 1, wobei die zylindrische Basis (1432) einen hohlen Innenraum hat, so dass eine oder mehrere Nahtmaterialpackungen (1430) mit abnehmendem Durchmesser in einer anderen Nahtmaterialpackung eingeschachtelt werden können.

4. Packung (1430) nach Anspruch 1, wobei der erste Nadelpark (1436) auf dem ersten Ende (1434) angeordnet ist und der zweite Nadelpark auf dem zweiten Ende (1435) angeordnet ist.

5. Packung (1430) nach Anspruch 1, wobei die zylindrische Basis (1432) zwei spiralförmige Kanäle (1431, 1433) umfasst, die in einer Doppelspiralbeziehung angeordnet sind.

6. Packung (1430) nach Anspruch 1, ferner umfassend eine Abdeckung (1466), die über die zylindrische Basis (1432) passt und die Außenfläche der zylindrischen Basis abdeckt.

7. Packung (1430) nach Anspruch 6, wobei die Abdeckung (1466) von der zylindrischen Basis abgestreift werden kann.

8. Packung (1430) nach Anspruch 6, wobei die Abdeckung (1466) geschlitzt und um die zylindrische Basis (1432) drehbar ist, um das Entfernen des Nahtmaterialfadens durch den geschlitzten Abschnitt der Abdeckung zu gestatten, wenn der Nahtmaterialfaden abgewickelt wird.

9. Packung (1430) nach Anspruch 6, wobei die Abdeckung (1466) in der Lage ist, von dem Nahtmaterialfaden gespalten zu werden, wenn der Nahtmaterialfaden abgewickelt wird.

## Revendications

1. Emballage de suture (1430) apte à contenir au moins une suture présentant un premier et un second segments, et une première aiguille qui s'étend à partir de l'extrémité du premier segment et une seconde aiguille qui s'étend à partir de l'extrémité du second segment, l'emballage de suture comprenant:
une base cylindrique (1432);
ladite base cylindrique présentant une première extrémité (1434) et une seconde extrémité (1435);
un premier porte-aiguille (1436) positionné sur une parmi ladite première extrémité et ladite seconde extrémité, et un second porte-aiguille (1437) positionné sur une parmi ladite première extrémité et ladite seconde extrémité;
ladite base cylindrique étant apte à avoir la suture enroulée autour de ladite base cylindrique;
ledit premier porte-aiguille étant apte à retenir la première aiguille; et
ledit second porte-aiguille étant apte à retenir la seconde aiguille,
**caractérisé en ce que** ladite base cylindrique comprend au moins un canal hélicoïdal (1433) qui établit une communication entre ladite première et ladite seconde extrémités et qui est déployé autour de ladite base cylindrique,
dans lequel ledit canal et lesdits premier et second porte-aiguilles sont aptes à retenir ensemble ladite suture de telle sorte que, lorsque la suture est emballée, ladite suture soit retenue de façon amovible dans ledit premier porte-aiguille et que ladite seconde aiguille soit retenue de façon amovible dans ledit second porte-aiguille afin de produire une tension,
ou dans lequel des attaches ouvrables sont prévues le long dudit canal hélicoïdal pour retenir ladite suture,
ou dans lequel un adhésif ou un gel amovible est prévu de façon continue ou intermittente le long dudit canal hélicoïdal pour retenir ladite suture,
de telle sorte que, lorsque la suture est emballée, le premier segment de ladite suture soit séparé dudit second segment de ladite suture.

2. Emballage (1430) selon la revendication 1, dans lequel ledit au moins un canal (1433) est une rainure en forme de v.

3. Emballage (1430) selon la revendication 1, dans lequel ladite base cylindrique (1432) présente un espace intérieur creux de telle sorte qu'un ou plusieurs emballage(s) de suture (1430) de diamètre décroissant puisse(nt) être emboîté(s) à l'intérieur d'un autre emballage de suture.

4. Emballage (1430) selon la revendication 1, dans lequel ledit premier porte-aiguille (1436) est disposé sur ladite première extrémité (1434), et ledit second porte-aiguille est disposé sur ladite seconde extrémité (1435).

5. Emballage (1430) selon la revendication 1, dans lequel ladite base cylindrique (1432) comprend deux canaux hélicoïdaux (1431, 1433) qui sont agencés dans une relation en double hélice.

6. Emballage (1430) selon la revendication 1, comprenant en outre une coiffe (1466) qui s'ajuste sur la base cylindrique (1432) et qui couvre la surface extérieure de ladite base cylindrique.

7. Emballage (1430) selon la revendication 6, dans lequel ladite coiffe (1466) peut être ôtée de ladite base cylindrique par glissement.

8. Emballage (1430) selon la revendication 6, dans lequel ladite coiffe (1466) est fendue et rotative autour de ladite base cylindrique (1432) de manière à permettre l'enlèvement de ladite suture à travers la partie fendue de ladite coiffe lorsque ladite suture est déroulée.

9. Emballage (1430) selon la revendication 6, dans lequel ladite coiffe (1466) peut être scindée par ladite suture lorsque ladite suture est déroulée.
